# EUROPEAN PATENT APPLICATION

(11) **EP 4 239 638 A1**
(43) Date of publication of application: **06.09.2023**
(21) Application number: 22160023.2
(22) Date of filing: 03.03.2022
(51) Int. Cl.: G16B 30/10

(54) **METHOD FOR DETERMINING VIRAL CONTAMINATION**

(71) Applicant: Ares Trading S.A., 1170 Aubonne (CH)
(72) Inventor: OLGIATI, Simone, 10010 Colleretto Giacosa (IT); CUCURACHI, Marco, 10010 Colleretto Giacosa (IT); LEMBO, Antonio, 10010 Colleretto Giacosa (IT)
(74) Representative: Merck Serono S.A. Intellectual Property

(57) **Abstract**

Described herein is a bioinformatic method for determining the presence of viral contamination in a sample and if such viral contamination is present the identification of the type(s) of contamination. The method uses high throughput sequencing techniques on DNA and/or RNA present in a sample. The methods described herein facilitate in-process control and r processing for release of batches, cell banks, bulk harvest and raw materials.

## Description

### Field of the Invention

The present invention relates to a bioinformatic method for determining the presence of a viral contamination in a sample. Particularly, the present invention relates to determining the type of viral contamination where such viral contamination is present. Such method can be used in various applications in which the absence of viral contamination is a critical quality attribute either for the final product or during any intermediate steps in or as raw material for a production process.

### Background

Viral contaminations, or adventitious contaminations, can occur in any environment and in any type of materials. Such materials can be for example a final or intermediate product in a process producing a biomolecule of interest or in raw materials for a production process to produce an active pharmaceutical agent or a cell bank.

The presence of harmful viral agents should be avoided in many commercially available products. This is particularly true for any products that are for human or animal consumption such as foods or medications. For example, many health authorities around the world require strict control of production processes to minimize viral contaminations. In many instances such health authorities require the final product, particularly if the final product is a medical product, to be free of viral contaminations.

Analytical methods have been developed for determining viral contaminations and frequently involve the testing of the presence of a particular viral contaminant by exposing a sample (or multiple samples simultaneously) to a determinant for a suspected viral contaminant. For example, using a reporter antibody that recognizes a particular viral contaminant. Such analytical methods are laborious and require the availability of a good reporter determinant for the viral contaminant with a very low limited of detection. In addition, such methods are limited in that only the presence or absence of a suspected viral contamination is determined.

Thus, there is a need, for alternative detection methods for determining viral contaminants in a sample, which are less cumbersome and laborious and would determine the presence or absence of a viral contamination regardless of the nature of a suspected viral contaminant.

### Summary of the Invention

The current invention provides a solution to the above described problems by sequencing all DNA and RNA, if present, in a sample to be tested for viral contamination and comparing the resulting sequencing reads to a viral database. Such a method is independent from the nature or type of suspected viral contamination. The method is rapid and can be carried out using high throughput sequencing (HTS, also known as Next-Generation Sequencing or massive parallel sequencing or deep sequencing) and requires a single sample. Moreover, the method of the present invention can be automated in production processes for commercial production of biomolecules. In such production processes the method of the current invention can be utilized in in-process control. In-process control for determining viral contamination could improve the reliability of production processes and provide control and insights in where within the production process the viral contamination occurred. Another advantage of the method of the present invention is that if a viral contamination is detected the nature/type of viral contamination can be readily identified.

In one embodiment, the present invention provides a method for determining viral contamination in a sample wherein sequence data is obtained through HTS, the method comprising the steps of:
a. obtaining a plurality of reads of DNA fragments from total DNA and/or RNA of a sample,
b. alignment of sequencing reads against a viral database,
c. subtracting sequencing reads from nucleic acid fragments that do not have similarity with viral sequences, and
d. determine viral contamination and identity of such viral contamination in the sample of the biomolecule of interest when one or more of the remaining sequencing reads is aligned with a sequence in the viral database. In some embodiments, the viral database comprises viral sequences organized by genomes and viral families. For example, the viral families can be organized such that viruses of the same taxonomic family are grouped together.

In another embodiment of the present invention, the current invention provides a method for determining viral contamination of a sample in the production process of a biologic molecule of interest, where the method for determining viral contamination in a sample and wherein sequence data is obtained through HTS, the method comprises the steps of:
a. obtaining a plurality of reads of DNA fragments from total DNA and/or RNA of a sample comprising a biologic molecule of interest,
b1. subtracting sequencing reads from nucleic acid fragments that align against the host cell genome,
b2. alignment of sequencing reads against a viral database,
c. subtracting sequencing reads from nucleic acid fragments that do not have similarity with viral sequences, and
d. determine viral contamination and identity of such viral contamination in the sample of the biomolecule of interest when one or more of the remaining sequencing reads is aligned with a sequence in the viral database.

In yet another embodiment, the current invention provides a method for determining viral contamination in a sample wherein sequence data is obtained through HTS, the method comprising the steps of:
a. obtaining a plurality of reads of DNA fragments from total DNA and/or RNA of a sample,
b. alignment of sequencing reads against a viral database,
c. subtracting sequencing reads from nucleic acid fragments that do not have similarity with viral sequences,
d. calculating a set of sequencing coverage metrics for each viral genome sequence after alignment of the remaining sequencing reads against the viral database,
e. discard all viral genomes not surpassing one or more preset minimal sequence coverage metric values,
f. identify and report any viral families that present at least one candidate positive signal,
g. identify for reach reported family a virus with the most complete and intense signal, and
h. report both a list of positive viral families and a best match in each positive family determining viral contamination and identity of such viral contamination in the sample.

In another embodiment, the present invention provides a method of product release in or from a production process the method comprising;
a. determining the presence or absence of viral contamination in a sample according to a method as described in any of the foregoing embodiments, and
b. confirming product release in the absence of viral contamination or in the presence of viral contamination below a preset level of contamination.

### Brief Description of the Drawings

Figure 1: Shows a flow diagram of the method of the present invention.
Figure 2: Shows a flow diagram of the method wherein in a sample obtained from a production process for a biologic molecule of interest the method of the present invention includes a step wherein the sequencing reads that are aligned with the host cell genome are subtracted prior to alignment with the viral database.
Figure 3: show a representation of the calculation in the coverage metrics; Step 1 Mapped reads; Step 2: Genome partition in 100 bp bins; Step 3 positive (red) and negative (blue) bins count; Step 4: Application of 1kb Bins counting positives (green) and negatives (grey); Step 5: 1X Coverage % (1kb Bins) calculation.
Figure 4: Shows a representation for differences in the calculation of the Unmasked and Masked processes for the "1X Coverage % (1kb Bins)" metric.

### Detailed Description of the Invention

Reliable, easy to use (even as in-process control) and rapid analytical methods for determining viral contaminations are currently not available as described above. The present invention provides a solution wherein viral contamination and identity, if present, of such viral contamination can be determined. The method of the present invention utilizes high through put sequencing techniques wherein the sequencing reads are compared and aligned with a viral database. Such methods allow for a more rapid determination of viral contamination and simultaneously identify the viral contaminants if there are one or more such viral contaminants. The method of the present invention can be used to determine viral contaminants in a variety of products such as for example a final product of a production process or raw materials to be used in a production process but also intermediate products (such as for in-process control).

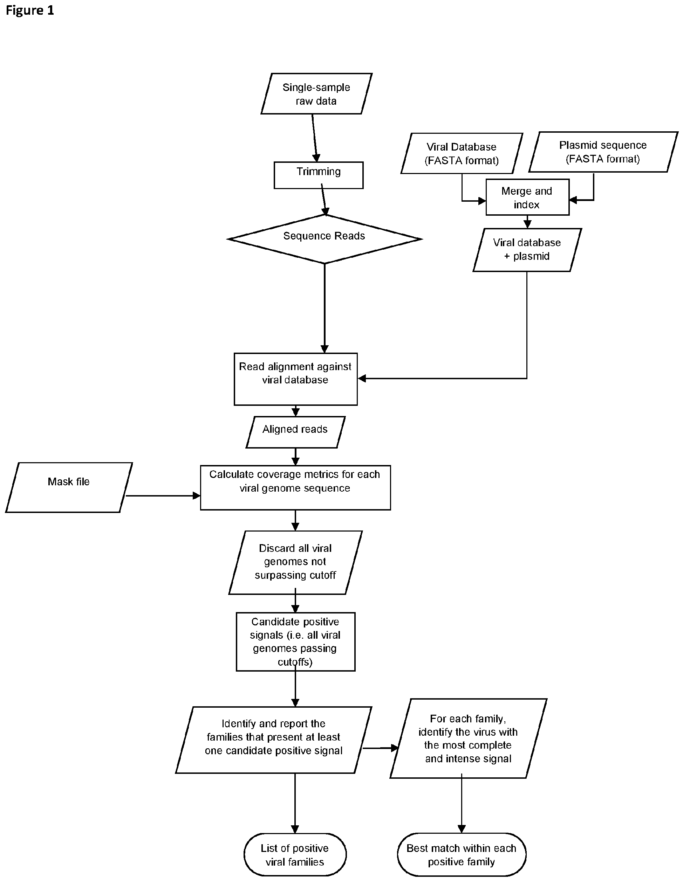

As such in one embodiment the present invention provides a method for determining viral contamination in a sample wherein sequence data is obtained through HTS, the method comprising the steps of:
a. obtaining a plurality of reads of DNA fragments from total DNA and/or RNA of a sample,
b. alignment of sequencing reads against a viral database,
c. subtracting sequencing reads from nucleic acid fragments that do not have similarity with viral sequences, and
d. determining viral contamination and identity of such viral contamination in the sample when one or more of the remaining sequencing reads is aligned with a sequence in the viral database. Figure 1, provides an overview of the method of the present invention in a flow-chart.

In the method of the present invention any high through-put sequencing (HTS) technique can be used, preferably the method uses short-read HTS methods.

The viral database for use in method of the present invention preferably comprises viral genome sequences organized by genome and viral families. The viral families (or taxonomic groups of other rank) are preferably organized such that viruses of the same taxonomic family are grouped together. In addition, such grouping together can also apply to sequences of segmented genomes which are grouped together in the viral database. As such the identity of the viral contamination, if there is any could be more readily determined.

Some sequencing reads obtained with the sequencing methods as used in the present invention can be aligned both with sequences in the viral database and are sequences that could be non-viral (having similarity with non-viral sequences). Taking into account that such sequencing reads that have similarity to non-viral sequences while at the same time align with sequences in the viral database a mask can be applied to the remaining sequences from step c in the method of the present invention. Such mask either fully subtracts such sequencing reads from the remaining sequencing reads from step c of the method or applies to such sequencing reads a discounted value. When calculating a set of sequence coverage metrics such sequencing reads which are discounted as a result of the mask would have a reduced value in the calculated coverage metrics as opposed to when no mask (unmasked coverage metrics) was applied.

The determination of viral contamination and identity, if any viral contamination is present, is preferably carried out by the steps of: a.) calculating a set of sequencing coverage metrics for each viral genome sequence after alignment of the remaining sequencing reads against the viral database, b.) discard all viral genomes not surpassing one or more preset minimal sequence coverage metric values, c.) identify and report any viral families that present at least one candidate positive signal, d.) identify for reach reported family a virus with the most complete and intense signal, and e.) report both a list of positive viral families and a best match in each positive family.

As described above such sequence coverage metrics can be calculated while excluding all viral genome regions (sequencing reads within a viral genome region) that overlap with sequences that have previously been observed in reference samples of a same biologic background. A reference sample of a same biologic background as described herein refers to when the sample for which viral contamination is tested has the same biologic background as a reference sample which does not contain any viral contamination. Such reference sample of a same biologic background is preferable a reference sample with known absence of viral contamination and that has been produced in a same production process using the same biologic material, for example host cell, as the sample for which the presence or absence of viral contamination is being tested. In addition, the biologic material referred to herein may be either from the host cell or could also refer to the plasmid sequence where the plasmid is introduced in the host cell for expressing the biologic molecule of interest. The plasmid contains some plasmid specific sequences and the sequence of the biologic molecule of interest. Also, such biologic material referred to herein may be sequence material related to a recombinant cell line under testing (for example in a cell bank or production process).

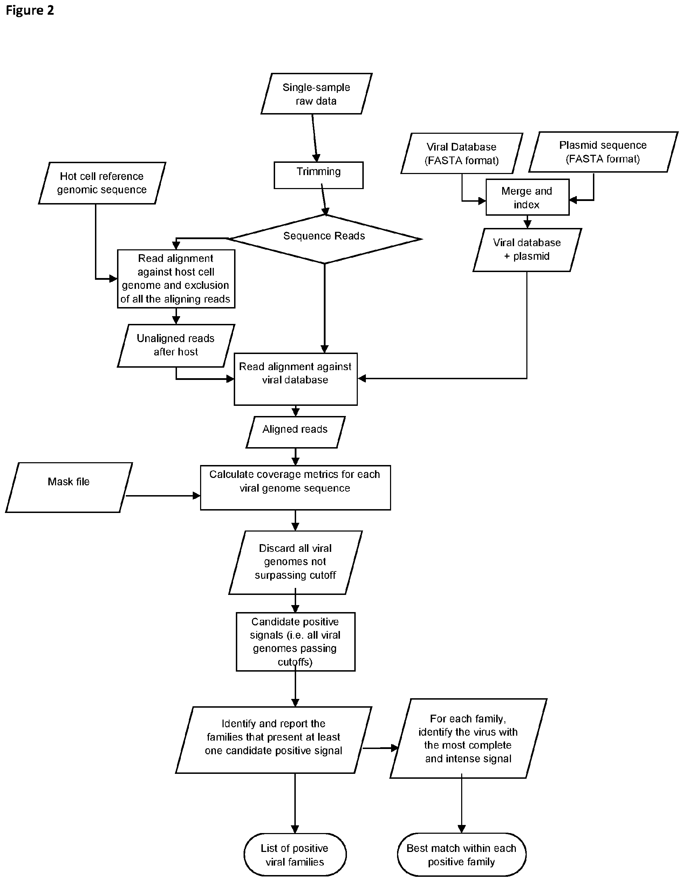

The method can be used for determining the presence of viral contamination in a variety of different samples such a final product, raw material or intermediate product in or for a production process. Preferably the production process is a production process for a biologic molecule. In such production process where the final product is a biologic molecule the process may use a host cell to express the biologic molecule. In processes wherein a host cell is used to express the biologic molecule, the process of the present invention includes the subtraction of any sequencing reads that are aligned with a host cell genome prior to alignment of the sequencing reads to the viral database as in step b of the method of the current invention. In the flow-chart of Figure 2 such method is shown wherein sequencing reads that are aligned with the host cell genome are subtracted.

The method of the present invention can be used in a method for product release. In such method for product release in or from a production process the method comprises determining the presence or absence of viral contamination in a sample by using the method of the current invention which includes the steps of:
a. obtaining a plurality of reads of DNA fragments from total DNA and/or RNA of a sample comprising,
b. alignment of sequencing reads against a viral database,
c. subtracting sequencing reads from nucleic acid fragments that do not have similarity with viral sequences, and
d. determine viral contamination and identity of such viral contamination in the sample when one or more of the remaining sequencing reads is aligned with a sequence in the viral database. Subsequently product release can be confirmed in the absence of viral or in the presence of viral contamination below a preset level of detection. In such process the product can be a final product, an intermediate product, for example a bulk harvest, or a raw material. The product in such a process or the raw material could be a cell bank, particularly when the process is a production process for preparing a biologic molecule.

The following examples are illustrative and are not meant to be limiting the scope of the invention.

### Example 1:

The raw data produced by NGS sequencers are analyzed and the method provides a determination on the presence or absence of viral contamination.

### 1. Generation of FASTQ files and trimming

The raw data generated by the are converted into FASTQ files. Where a single run includes data from different samples (multiplexed runs), in this initial step the reads are assigned to each sample.

After the generation of the FASTQs, the sequence of the adapters are removed from the reads in a processed called "trimming". This step is required to filter out reads with low quality and to "clean" the data, because part of the sequences generated during the sequencing process might contain adapters used for the sequencing itself.

Optionally, the pipeline can subsample the reads in order to use only part of the available reads for data analysis. This optional step can be used to assess the method performance at different levels of sequencing throughput (i.e. different number of sequencing reads generated).

### 2. host cell subtraction (which is optional)

To remove sequences derived from the cell genome, the method can align all the reads against the reference genomic sequence of the host cell using a sequence aligner. Then, all the reads aligning against the host genome are excluded from the analysis and only the unaligned reads are used for the subsequent steps. This step is optional but can be useful when a mask file is not available, or it can be used to further investigate positive samples, excluding false positives due to low specificity.

### 3. Alignment against reference viral database and plasmid sequence

The reads generated in step 1 (or step 2 in case the step host subtraction is carried out) are then aligned against a database including both the plasmid sequence as well as the viral database using an open source sequence aligner generating an intermediate alignment file. The resulting alignment file is further processed to make sure that i) secondary alignments (i.e. reads that align equally well to multiple locations) are treated as primary (i.e. considered in the downstream processing) and ii) alignments shorter than 75 base pair are discarded. At the end of this process an alignment file in BAM format is generated. This file contains various information including: i) Name of the read, ii) Location of the alignment on the reference database (with sequence name) iii) Quality of the alignment, iv) Quality of the read and v) name of the sequence of the reference genome.

### 4. Computation of coverage metrics

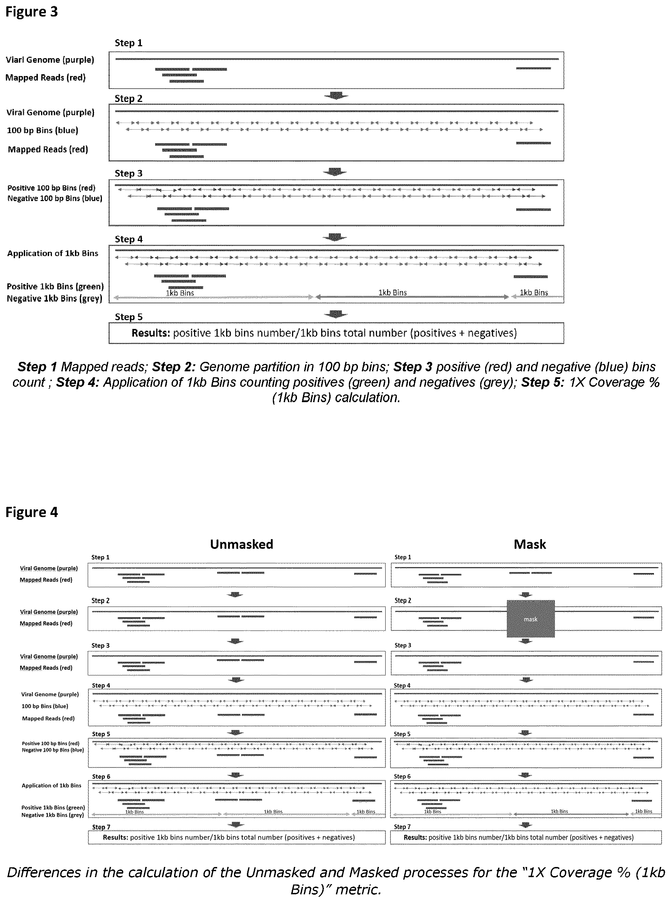

In this step the method disregards all the alignments against the plasmid sequence and calculates several coverage metrics for each viral genome.
- The "Mapping Reads" is the count of aligned reads against each viral reference genome sequence.
- The "1X Coverage %" is the ratio between the number of bases of the viral genome covered by at least one read and the total length of the genome (for fragmented genomes, this is the sum of the different genomic fragments). This coverage does not indicate whether the reads cover more or less uniformly the entire genomic sequence, but only the proportion of genome detected.
- The "3X Coverage %" is the ratio between the number of bases of the viral genome covered by at least three reads and the total length of the genome (for fragmented genomes, this is the sum of the different genomic fragments).
- The "1X Coverage % (1kb Bins)" takes into account the distribution of reads across the viral sequence. For all the viruses in the database, the method divides the genome into windows ("bins") of 100 base pairs (bp) overlapped by 50 bp. Then the number of positive bins (where at least one read was observed) and negative bins (no reads observed) are counted for each genome. Subsequently the method divides all the genomes into 1 Kbp bins and counts them as positive if they contain positive 100 bp bins and negative otherwise. At the end, the method calculates the "1X Coverage % (1kb Bins)" as the ratio between the number of positive 1 Kbp bins and the total number of 1 Kbp bins (positives + negatives) as shown in Figure 3.

All the four parameters are calculated for all the viruses 2 times, one counting the entire length of the viral sequences including in the database (metrics "unmasked") and a second time excluding all the viral regions that are described in the Mask file (metrics "masked"). See Fig. 4 for a more detailed description on the calculation of the "1X Coverage % (1kb Bins)".

### 5. Selection of candidate positive signals.

The coverage metrics calculated in the previous step are used to discriminate background noise from potential signals. Cutoff values preset or previously set (for example previously determined through empirical evidence) are used to exclude all the viral signals that do not pass the defined cutoffs, selecting the positive candidate signals.

### 6. Identification of positive viral groups and best match

Using virus details present in the database, the method determines which viral groups (e.g. taxonomic families) contains at least one candidate positive signals. These viral groups are added to the final report and the list of positive viral families constitutes the primary result of the method. In addition, for each positive viral group, the method identifies which viral genome is the closest match to the actual viral contaminant in the sample ("Best match"). For each positive viral group, the best match reported by the method is the virus with the highest 1X Coverage % (unmasked). In case of ties between two sequences, the method selects the signal with highest number of mapping reads (unmasked).

## Claims

1. A method for determining viral contamination in a sample wherein sequence data is obtained through high through-put sequencing (HTS), the method comprising the steps of:
a. obtaining a plurality of reads of DNA fragments from total DNA and/or RNA of a sample comprising,
b. alignment of sequencing reads against a viral database,
c. subtracting sequencing reads from nucleic acid fragments that do not have similarity with viral sequences, and
d. determine viral contamination and identity of such viral contamination in the sample when one or more of the remaining sequencing reads is aligned with a sequence in the viral database.

2. The method of claim 1, wherein the viral database comprises viral genome sequences organized by genomes and viral families.

3. The method of claim 2, wherein the viral families are organized such that viruses of the same taxonomic family are grouped.

4. The method of any of the preceding claims, wherein in the viral database sequences of segmented genomes are grouped.

5. The method of any of the preceding claims, wherein the plurality of sequencing reads are obtained by short-read HTS methods or long-read HTS methods.

6. The method of claim 5, wherein the plurality of sequencing reads is obtained by short-read HTS methods.

7. The method of any of the preceding claims, wherein step c) further comprises subtracting sequencing reads from nucleic acid fragments with similarity to background sequences that are non-viral and that have similarity to sequences that are viral.

8. The method of any of the preceding claims, wherein, when the sample is a sample from a production process comprising the use of a host cell, step b) is preceded by subtracting sequencing reads that align against the host cell genome.

9. The method of any of the preceding claims, wherein step d) comprises;
a. calculating a set of sequencing coverage metrics for each viral genome sequence after alignment of the remaining sequencing reads against the viral database,
b. discard all viral genomes not surpassing one or more preset minimal sequence coverage metric values,
c. identify and report any viral families that present at least one candidate positive signal,
d. identify for reach reported family a virus with the most complete and intense signal, and
e. report both a list of positive viral families and a best match in each positive family.

10. The method of claim 9, wherein each sequence coverage metric is calculated while excluding all viral genome regions that had been previously observed in reference samples with the same biological background.

11. A method of product release in or from a production process the method comprising;
a. determining the presence or absence of viral contamination in a sample according to the method of any of the preceding claims, and
b. confirming product release in the absence of viral contamination or in the presence of viral contamination below a preset level of contamination.

12. The method of claim 11, wherein the product release is selected from batch release, bulk harvest release in in-process control, cell bank release and raw materials release.

13. The method of claim 12, wherein the batch release is in a biologic production process for a biologic molecule.

14. The method of any one of claims 11-13, wherein when the product release identifies more than one viral contaminant the method further comprises determining a ranking in major and/or minor viral contaminants.
